(19) Europäisches Patentamt
European Patent Office
Office européen des brevets

(11) EP 1 042 458 B1

(12) EUROPEAN PATENT SPECIFICATION

(45) Date of publication and mention of the grant of the patent:
**12.11.2003 Bulletin 2003/46**

(21) Application number: **98959787.7**

(22) Date of filing: **16.12.1998**

(51) Int Cl.[7]: **C12N 11/02**, C12N 11/14

(86) International application number:
**PCT/DK98/00554**

(87) International publication number:
**WO 99/033964 (08.07.1999 Gazette 1999/27)**

(54) **A PROCESS FOR IMMOBILISATION OF ENZYMES**

VERFAHREN ZUR IMMOBILISIERUNG VON ENZYMEN

PROCEDE SERVANT A IMMOBILISER DES ENZYMES

(84) Designated Contracting States:
**AT BE CH DE DK ES FI FR GB GR IE IT LI NL PT SE**

(30) Priority: **23.12.1997 DK 152797**

(43) Date of publication of application:
**11.10.2000 Bulletin 2000/41**

(73) Proprietor: **Novozymes A/S**
**2880 Bagsvaerd (DK)**

(72) Inventors:
- **CHRISTENSEN, Morten, Würtz**
**DK-2800 Lyngby (DK)**
- **KIRK, Ole**
**DK-2830 Virum (DK)**
- **PEDERSEN, Christian**
**DK-2610 R dovre (DK)**

(56) References cited:
**EP-A1- 0 140 542**
**WO-A1-95/22606**
**WO-A2-94/26883**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 042 458 B1

Printed by Jouve, 75001 PARIS (FR)

## Description

### FIELD OF THE INVENTION

The invention relates to a process for producing an immobilized enzyme preparation for use in a mainly organic medium essentially devoid of free water, and use of the immobilized enzyme preparation for organic synthesis.

### BACKGROUND OF THE INVENTION

Immobilized enzymes are known to be used for organic synthesis.

The most commonly immobilized enzymes are lipases used for esterification reactions in mainly organic media essentially devoid of free water.

EP 140542 B2 describes a process, wherein an enzyme containing liquid is brought in contact with a weak anion exchange resin carrier by dispersing the carrier in the liquid and mixing by stirring with a magnetic stirrer, whereby the enzyme is immobilized on the carrier. The immobilization is subsequently followed by vacuum drying of the enzyme-carrier.

WO 95/22606 describes a process, wherein an enzyme containing liquid is brought in contact with porous silica carrier by atomizing the liquid onto the carrier in a mixer, subsequently followed by drying overnight a ambient conditions

In industrial immobilization processes described in prior art, the carrier or support material is placed in a column shaped adsorption vessel and an enzyme containing liquid is recirculated until sufficient adsorption of the enzyme on the carrier has been obtained. Following the adsorption step the column is emptied by manually shoveling the enzyme-carrier product into trays. The product is then dried by placing the trays under vacuum at room temperature for a period of 14-16 hours.

WO 94/26883 describes a process for producing dust-free enzyme granules by absorbing the enzyme on a porous material, said material including NaCl, Soda, and silica, and optionally coating the product with a protective outer layer. Generally immobilization of enzymes should not be compared with granulation of enzymes as granulation serves a completely different purpose, viz. to provide a preferably non-dusting delivery material from which an enzyme may be delivered to an aqueous solution by disintegration of the granule and/or dissolution of the enzyme in the aqueous phase. Enzyme immobilization concerns immobilizing an enzyme product on a carrier on which the enzyme is fixed and yet functional and for which the enzyme is not liberated to the. solvent to which it is applied.

Immobilization processes known to the art are limited in capacity as it involves laborious and manual steps and require heavy equipment investments (e.g. vacuum rooms), which, in turn, means inflexible production and expensive products.

### BRIEF DESCRIPTION OF THE DRAWINGS

Figure 1 shows an example of acidolysis catalyzed by a lipase, wherein a first reactant (reactant 1) is reacted with a second reactant (reactant 2), e.g. a free fatty acid, the substituents R1 and R2 being exchanged in said reaction. R1 and R2 may as an example be:

$$R1 = -(CH_2)_7-\underset{H}{C}=\underset{H}{C}-(CH_2)_7-CH_3$$

$$R2 = -(CH_2)_{16}-CH_3$$

Figure 2 shows an example of inter-esterification catalyzed by a lipase, wherein a first reactant (reactant 1), e.g. a triglyceride is reacted with a second reactant (reactant 2), e.g. another triglyceride, the substituents R1 and R2 being exchanged in said reaction. R1 and R2 may as an example be:

$$R1 = -(CH_2)_7-CH=CH-(CH_2)_7-CH_3$$

$$R2 = -(CH_2)_{16}-CH_3$$

Figure 3 shows an example of alcoholysis catalyzed by a lipase, wherein a first reactant (reactant 1), e.g. a triglyceride is reacted with a second reactant (reactant 2), e.g. an alcohol, the substituents R1 and R2 being exchanged in said reaction. R1 and R2 may as an example be:

$$R1 = -(CH_2)_7-CH=CH-(CH_2)_7-CH_3$$

$$R2 = -(CH_2)_{16}-CH_3$$

## SUMMARY OF THE INVENTION

The present invention provides alternative processes for industrial immobilization of enzymes, which significantly increases capacity and reduces labor costs, by means of standard multi-purpose process equipment.

Thus the invention provides processes for producing an immobilized enzyme preparation for use in a mainly organic medium essentially devoid of free water, which in a first aspect comprises:

a) fluidising a particulate porous carrier in a fluid bed,

b) introducing an enzyme containing liquid medium by atomization into the fluid bed, so as to adsorb the enzyme on the carrier, and

c) removing volatile components of the liquid medium from the carrier in the fluidized bed.

In a second aspect the processes comprise:

a) contacting an enzyme containing liquid medium with a particulate porous carrier with a substantially hydrophobic surface, so as to adsorb the enzyme on the carrier, and

b) introducing a particulate hygroscopic substance, so as to suppress agglomeration of the carrier,

c) removing volatile components of the liquid medium and the hygroscopic substance from said product in a fluidized bed.

Finally in a third aspect the processes comprise:

a) introducing an enzyme containing liquid medium by atomization onto a particulate porous carrier with a substantially hydrophilic surface, so as to adsorb the enzyme on the carrier, wherein the liquid is introduced in an amount such that substantially no agglomeration of the carrier occurs and

b) removing volatile components of the liquid medium from said product in a fluidized bed.

## DETAILED DESCRIPTION OF THE INVENTION

### The Carrier

In the embodiment of the invention the carrier is a particulate porous material. The particles may suitably be of a diametrical size of 200-1000 μm, preferably 400-700 μm; have a surface area of 20-1000 $m^2/g$, preferably 100-700 $m^2/g$ and have a pore size of 10-500 nm, preferably 100-300 nm.

The carrier particles may comprise inorganic, organic or both inorganic and organic material. Said carrier may further have a hydrophilic or hydrophobic surface.

In a first embodiment of the invention the carrier particles comprise an inorganic material with a substantially hydrophilic surface, which is essentially insoluble in hydrophilic or hydrophobic liquids or mixtures thereof. Preferred carriers may be based on silicas (e.g. Celite from Manville, USA), zeolites (e.g. Wessalith MS330 from Degussa, Germany), aluminas, ceramics (e.g. as disclosed in Yoshihiko Hirose et Al. (Proceedings from 3$^{rd}$ International Symposium on Biocatalysis and Biotransformations, La Grande Motte, France, 1997, p238)and kaolins (e.g. kaolin's subjected to acid, hydrothermal and baking treatment as disclosed in US Patent No. 5,614,401).

In a second embodiment of the invention the carrier particles comprise a hydrophilic inorganic material as described in the first embodiment coated with organic moieties thus having a substantially hydrophobic surface, e.g. as described in JP 09000257-A, wherein an acid treated kaolin carrier is coated with N-phenyl-gamma-aminopropyltrimethoxysilane. Further carriers are described in JP 08126489-A, wherein a water insoluble carrier is coated with a polymer forming a disulphide linkage with enzymes. A third type of carriers is described in *Biotechnology Techniques vol. 3 No 5 345-348*, wherein a ceramic carrier is coated with polyethylene amine, polyethylene imine or 3-aminopropyltriethoxysilane, all three surface types allowing an enzyme to be covalently bound via glutaraldehyd coupling.

In a third embodiment of the invention the carrier particles comprise an organic polymer resin with a substantially hydrophobic surface. The resin may be an adsorbent resin, preferably a polyacrylate, a polymethacrylate (e.g. polymethyl methacrylate), polystyrene cross-linked with divinylbenzene, polyurethane or polypropylene or the resin may be an ion exchange resin, preferably an anion exchange resin, e.g. a weakly basic anion exchange resin. A preferred anion exchange resin is a phenolic type Duolite resin from Rohm & Haas.

Further the carrier may be made from regenerated chitosan as disclosed in DE 4429018-A.

**The Enzyme**

The enzyme to be immobilized according to the invention may be any enzyme suitable for use in media essentially devoid of free water. The most commonly used enzymes are lipases and in a specific embodiment of the invention the lipase may be derived from a strain of the genus *Humicola* (also known as *Thermomyces*), *Pseudomonas, Candida,* or *Rhizomucor,* preferably the species *H. lanuginosa* (also known as *Thermomyces lanuginosa* as described in US 4,810,414 and EP 305216 which are hereby included by reference), *C. antarctica* or *R. miehei*.

Further the lipase may be positionally site specific (i.e. 1,3 specific) or non-specific, upon interaction with triglycerides as substrates.

The enzyme may further be covalently cross-linked by glutaraldehyde treatment during the immobilization process.

**The enzyme containing liquid medium**

The enzyme containing liquid medium is a hydrophilic medium, preferably aqueous. It may thus contain more than 20% water, preferably more than 40% water, more preferably more than 50% water, more preferably more than 60% water, more preferably more than 70% water, more preferably more than 80% water, more preferably more than 90% water, e.g. more than 95% water. It may contain other organic or biological matter. Thus it may be a fermentation broth or an enzyme concentrate solution obtainable by purifying a fermentation broth by e.g. ultra filtration or by protein precipitation, separation and re-dissolution in an other aqueous medium. It may further be substantially pure enzyme dissolved in an aqueous medium. In a special embodiment of the invention the enzyme containing aqueous liquid has not been subjected to costly processing steps prior to immobilization to remove water such as evaporation nor has it been subjected to addition of non aqueous solvents, e.g. organic solvents such as alcohols, e.g. (poly)ethylene glycol and/or (poly) propylene glycol.

**The immobilization process**

<u>Immobilization of enzyme on carriers with a hydrophilic surface</u>

Without being bound to the theory it is contemplated that immobilization of enzyme on carriers having a substantially hydrophilic surface involves no adsorption of the enzyme, but is a deposition of enzyme in surface pores as a result of removal of the liquid, in which the enzyme is dissolved.

***i.*** In one embodiment of the invention the immobilization of enzyme on a carrier having a substantially hydrophilic surface may thus be conducted in a standard mixing equipment (e.g. Lödiger, Germany), wherein an enzyme containing liquid is introduced by atomization to the dry porous and particulate carrier during mixing, e.g. using a nebulizer connected to a pump (e.g. a standard peristaltic Watson-Marlow pump).

The liquid should be added in such amounts that substantially no agglomeration of the carrier occurs, thus

enabling the subsequent drying of the enzyme-carrier product by fluidising said product in a standard fluid bed equipment, e.g. a Uni-Glatt fluidized bed apparatus (Glatt, Germany), thereby removing volatile components.

***ii.*** In a second embodiment of the invention the immobilization of enzyme on a carrier having a substantially hydrophilic surface may alternatively be conducted in a standard fluid bed equipment, e.g. a Uni-Glatt fluidized bed apparatus (Glatt, Germany), wherein the dry porous and particulate carrier is fluidized and an enzyme containing liquid at ambient temperature is introduced by atomization to the fluidized carrier, e.g. using a nebulizer connected to a pump (e.g. a standard peristaltic Watson-Marlow pump). In this embodiment immobilization and drying are conducted simultaneously.

Immobilization on carriers with a hydrophobic surface

Without being bound to the theory it is contemplated that immobilization of enzyme on carriers having a substantially hydrophobic surface involves adsorption of the enzyme on the surface. The immobilization may be enabled by the enzyme forming hydrogen bonds, ionic bonds or covalent bonds with moieties in the surface.

***iii.*** In a third embodiment of the invention the immobilization of enzyme on a carrier having a substantially hydrophobic surface may thus be conducted in a standard mixing equipment, wherein an enzyme containing liquid is introduced to the dry porous and particulate carrier in amount thus forming a paste or a slurry. The paste or slurry is mixed for a period of time in which the enzyme is adsorbed. Following the adsorption step a hygroscopic particulate substance of a particle size smaller than the carrier is introduced to the slurry or paste. Said substance substantially prevents agglomeration of the enzyme-carrier by adsorption of excess liquid, thereby enabling the subsequent drying of the enzyme-carrier product by fluidising said product in a standard fluid bed equipment, e.g. a Uni-Glatt fluidized bed apparatus (Glatt, Germany), thereby removing volatile components and if necessary the hygroscopic substance. The hygroscopic substance may be any particulate fine ground hydrophilic components capable of absorbing excess liquid such as silicas (e.g. Hyper Flow Celite), kaolin, aluminas, zeolites or ceramics. Removal of the hygroscopic substance may be achieved by inserting a filter at the top of the fluidized bed with a suitable pore size which will allow the hygroscopic substance to pass but will retain the enzyme-carrier. A pore size of 100-900 μm, preferably 200-400 μm may be employed.

**iv.** In a fourth embodiment of the invention the immobilization of enzyme on a carrier having a substantially hydrophobic surface may alternatively be conducted in a standard fluid bed equipment, e.g. a Uni-Glatt fluidized bed apparatus (Glatt, Germany), wherein the dry porous and particulate carrier is fluidized and an enzyme containing liquid at ambient temperature is introduced by atomization to the fluidized carrier, e.g. using a nebulizer connected to a pump (e.g. a standard peristaltic Watson-Marlow pump). In this embodiment immobilization and drying are conducted simultaneously.

Common features for *i* and *iii* mixing step

Immobilizing the enzyme on the carrier in a mixer may suitably be conducted at ambient temperature. Mixing times may for this size of equipment suitably be 5-60 minutes, preferably 10-30 minutes.

Common features for *i, ii, iii* and *iv* fluid bed step

A suitable air inlet flow in the fluid bed equipment will depend on the size and density of the enzyme-carrier, the amount of carrier and the fluid bed equipment. Further the air inlet flow has an upper limit, as the flow should be sufficient to keep the enzyme-carrier fluidized, but not so powerful as to "blow off" the enzyme-carrier.

Suitable temperatures of the inlet air for removing volatile components will primarily depend of the thermal stability of the enzyme (the inactivation temperature). The temperature may be 40-90°C, preferably 50-70°C, e.g. 60°C. A higher temperature provides shorter immobilization and drying times.

Further, time consumption for immobilization and/or drying of the enzyme-carrier when equipment, air inlet flow and air temperature are fixed will depend on the quantity of enzyme-carrier. The immobilization/drying process may be monitored by measuring the air inlet temperature and the air outlet temperature. While the enzyme-carrier is moist the outlet temperature is lower than the inlet temperature due to the heat absorption and evaporation of volatile components. Typically a steady state evaporation occurs during the immobilization/drying process where the outlet temperature stabilizes on a temperature lower than the inlet temperature indicating that evaporation of volatile components (i.e. heat absorption) occurs at a constant rate. At the end of the immobilization/drying process the outlet temperature begins to raise and approaching the inlet temperature indicating that the heat absorption has decreased

and thus the moisture of the enzyme-carrier has been removed. Using a fluid bed for immobilization and drying simultaneously the drying process will occur for as long as the enzyme containing liquid is atomized into the fluid bed, and may suitably be extended for 5-30 minutes after inlet of the enzyme containing liquid has ended.

An important aspect of the invention is that the immobilization processes can be easily scaled up by applying other larger standard equipment. Thus the equipment setting ranges given *vide supra* may be adjusted to optimize larger scale equipment.

**Uses of immobilized enzyme preparation**

Immobilized enzyme prepared in context of the invention may be used for hydrolysis, synthesis or modification of organic substances in a medium essentially devoid of free water. Said substances may be comprised in food products like plant or animal oils/fats.

Accordingly the invention encompasses a process for enzymatic modification of an organic compound comprising contacting in a reaction medium essentially devoid of free water said organic compound with an immobilized enzyme produced according to the invention.

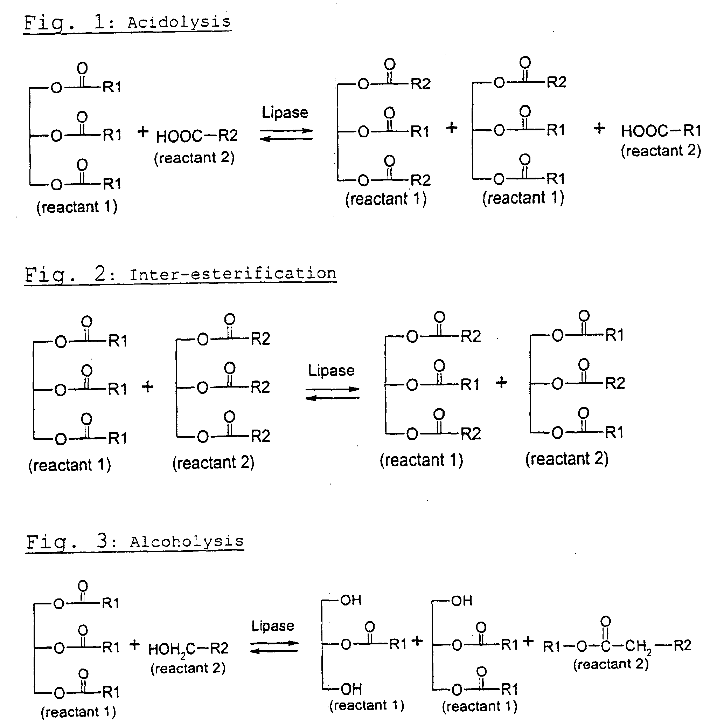

In a preferred embodiment of the invention an immobilized lipase enzyme, e.g. a 1,3 specific lipase, is used for an trans-esterification process in a medium essentially devoid of free water. The trans-esterification may be used for fatty acid substitution, comprising contacting a first reactant and a second reactant with said immobilized lipase by which a substitution reaction occurs, e.g. as shown in Figures 1-3.

The first reactant may be a fatty acid ester, preferably a triglyceride or a mixture of triglycerides.

The second reactant may be another fatty acid ester different from the first reactant, preferably a triglyceride or a mixture of triglycerides. Further the second reactant may be an alcohol or a free fatty acid.

The medium in this preferred embodiment of the invention comprises an organic solvent, or it may consist essentially of triglycerides.

Said use of the invention may be applied in production of food products e.g. margarine or cocoa-butter substitutes.

The invention is illustrated by the following non-limiting examples.

**EXAMPLES**

<u>Lipase assay:</u>

The lipolytic activity may be determined using tributyrine as substrate. This method is based on the hydrolysis of tributyrine by the enzyme, and the alkali consumption is registered as a function of time.

One Lipase Unit (LU) is defined as the amount of enzyme which, under standard conditions (i.e. at 30.0°C; pH 7.0; with Gum Arabic as emulsifier and tributyrine as substrate) liberates 1 mmole titrable butyric acid per minute.

A folder AF 95/5 describing this analytical method in more detail is available upon request to Novo Nordisk A/S, Denmark, which folder is hereby included by reference.

<u>Trans-esterification assay</u>

a)200 mg Trilaurin (Fluka) and 571 mg of myristic acid (8 molar equivalents) of myristic acid (Merck) was dissolved in 20 ml heptane. 3 ml saturated NaCl solution was added and the mixture was stirred in a closed bottle for 24 hours at ambient temperature.

b)The immobilized enzyme (50 mg) was water equilibrated in a dissicator (hermetically closed vessel) for 24 hours, using gas phase equilibrium with a saturated NaCl solution (water activity = 0.75).

c)At T = 0 minutes the water equilibrated immobilized enzyme and substrate was mixed in a closed bottle, which was placed in a shaking bath at 40°C. 100 μl samples were withdrawn from the closed bottle using a syringe at T = 0, 10, 20, 30, 40, 50 and 60 minutes. The samples were diluted (1:5 vol:vol) with a 50/50 (% v/v) mixture of acetone/acetonitrile and analyzed on a HPLC system.

Analysis on the HPLC system:

d)The HPLC system was equipped with a LiChrosphere 100 RPC18 endcapped 5 μm (125 × 4 mm) column (Merck). A 50/50 (% v/v) isocratic acetonitrile/acetone solution was selected as the mobile phase with a flow of 1 ml/minute.

e)20 µl of sample was injected and the formed products (1,2-dilauroyl-3-myristoyl-glycerol. (product 1) and 1,3-dimyristoyl-2-lauroyl-glycerol (product 2)) were measured by evaporative light scattering detection (Sedex 55, Sedere, France) at 2 bar pressure and a temperature at 30°C.

f)The amounts of formed products were estimated by comparing sample measurements to external standard curves of 1,2-dilauroyl-3-myristoyl-glycerol and 1,3-dimyristoyl-2-lauroyl-glycerol.

The rate of the trans-esterification process may be calculated in units, where 1 unit is defined as 1 umole myristic acid incorporated in trilaurin per minute.
Alternatively the effect of the inter-esterification process may be stated in % conversion of trilaurin to product 1 and product 2, which is calculated by:

$$\%\ \text{Conversion}_{T=i} = \left[\frac{\text{mole product 1} + \text{mole product 2}}{\text{mole trilaurin at } T = 0}\right]_{T=i} \cdot 100\%$$

where *i* indicates at which time the sample is withdrawn from the incubation bottle (step c).

<u>Example 1</u>

Lipase adsorption onto zeolite based carrier in fluid bed with simultaneous removal/evaporation of volatile liquids

400 g of a solution of *Humicola lanuginosa* lipase (693 kLU/ml) was atomized onto 1 kg zeolite (Wessalith MS330; 0.5-0.9mm; Degussa, Germany) using a two-way nebulizer in a Uni-Glatt (Glatt, Germany) fluidized bed apparatus. The lipase solution was applied via an peristaltic pump (Watson-Marlow). Inlet air temperature was 60°C and product temperature was 40°C with an air flow at 100 m3/hours. After the immobilization was finished the product was dried for an additional 5 min in the fluid bed.

The immobilization process was tested on the inter-esterification assay, which measured 53% conversion of trilaurin after T = 24 hours

<u>Example 2</u>

Lipase adsorption onto silica based carrier in fluid bed with simultaneous removal/evaporation of volatile liquids.

94 g of a solution of *Humicola lanuginosa* lipase (693 kLU/ml) fermentation solution was diluted with 100 g of demineralized water and atomized onto 200 g of Celite R648 (Manville, USA) in a Uni-Glatt (Glatt, Germany) fluidized bed apparatus, using a two-way nebulizer (made in-house). The lipase solution was applied via an peristaltic pump (Watson-Marlow)(flow rate 238 g/hour). Inlet air temperature and product temperature were identical with those indicated in example 1. After the immobilization was finished the product was dried for an additional 5 min in the fluid bed.

The immobilization process was tested on the inter-esterification assay, which measured 12% conversion of trilaurin after T = 24 hours

<u>Example 3</u>

Lipase adsorption onto adsorbent resin in mixer and subsequent drying in fluid bed using Hyper Flow Celite (HFC) as drying aid.

94 g of a solution of *Humicola lanuginosa* lipase (693 kLU/ml) was diluted with 260 g demineralized water. The solution was added to 250 g adsorbent resin (a macro-porous divinylbenzene cross-linked polystyrene, Purolite AP 1090; from Purolite, UK) in a 5 l mixer (Lödiger, Germany). The suspension was mixed for 20 minutes at ambient temperature and with RPM of 30. 200 g Hyper Flow Celite (HFC) was added to absorb residual liquid enabling the mixture to be fluidized in the Uni-Glatt apparatus. Using the same conditions as described in example 1, the mixture was dried for 20 min. In this period, the HFC was separated from the adsorbent resin using a 300 µm filter on the top of the fluid bed to retain the resin particles while HFC was blown off.

Activity of product:

The rate for T = 0 - 60 minutes was measured to 3,9 U/g product.

Example 4

Lipase adsorption onto adsorbent resin in fluid bed with simultaneous drying of volatile liquids.

94 g of a solution of Humicola lanuginosa lipase (693 kLU/ml) was diluted with 200 g of demineralized water and atomized onto 200 g of adsorbent resin (macro-porous divinylbenzene cross-linked polystyrene, Purolite AP 1090; from Purolite, UK) in a Uni-Glatt (Glatt, Germany) fluidized bed apparatus, using a two-way nebulizer. The lipase solution was applied via an peristaltic pump (Watson-Marlow) (flow rate 238 g/hour). Inlet air temperature and product temperature were 50°C and 30°C, respectively. After the immobilization was finished the product was dried for an additional 5 min in the fluid bed (flow rate 100 m3/hour).

The immobilization process was tested on the inter-esterification assay, which measured 36% conversion of trilaurin after T = 24 hours.

Example 5

Lipase adsorption onto a kaolin based carrier in fluid bed with simultaneous drying of volatile liquids.

94 g of a solution of Humicola lanuginosa lipase (693 kLU/ml) was diluted with 300 g of demineralized water and atomized onto 200 g of kaolin carrier (BIOFIX SC (500-250); from ECC, UK) in a Uni-Glatt (Glatt, Germany) fluidized bed apparatus, using a two-way nebulizer (made in-house). The lipase solution was applied via an peristaltic pump (Watson-Marlow) (flow rate 238 g/hour). Inlet air temperature and product temperature were 50°C and 30°C, respectively. After the immobilization was finished the product was dried for an additional 5 min in the fluid bed (flow rate 100 m3/hour).

The immobilization process was tested on the inter-esterification assay, which measured 34% conversion of trilaurin after T = 24 hours.

## Claims

**1.** A process for producing an immobilized enzyme preparation for use in a mainly organic medium devoid of free water, comprising:

a) Fluidising a particulate porous carrier having a surface area of 20-1000 $m^2/g$, a particle size of 200-1000 μm and comprises material selected from:

(1) an inorganic material, which is essentially insoluble in hydrophilic or hydrophobic liquids or mixtures thereof, having a hydrophilic surface,
(2) an inorganic material, which is essentially insoluble in hydrophilic or hydrophobic liquids or mixtures thereof, coated with organic moieties so as to provide a substantially hydrophobic surface or
(3) an organic polymer resin,

in a fluid bed.

b) introducing an enzyme containing liquid medium by atomization into the fluid bed, so as to fixate the enzyme on the carrier, and

c) removing volatile components of the liquid medium from the carrier in the fluidised bed.

**2.** A process for producing an immobilized enzyme preparation for use in a mainly organic medium devoid of free water comprising:

a) contacting an enzyme containing liquid medium with a particulate porous carrier which is essentially insoluble in hydrophilic or hydrophobic liquids or mixtures thereof, having a surface area of 20-1000 $m^2/g$, a particle size of 200-1000 μm, and a hydrophobic surface, so as to adsorb the enzyme on the carrier, and

b) introducing a hygroscopic substance, so as to suppress agglomeration of the carrier by absorbing excess liquid, and

c) removing volatile components of the liquid medium and the hygroscopic substance from said product in a fluidized bed.

3. The process of claim 1, wherein the liquid is introduced in an amount such that substantially no agglomeration of the carrier occurs.

4. The process of claim 1 wherein the inorganic material of step (1) is selected from the group consisting of silicas, zeolites, aluminas and kaolins.

5. The process of claim 1, wherein the inorganic material of step (2) is selected from the group consisting of silicas, zeolites, aluminas and kaolins coated with organic moieties so as to provide a hydrophobic surface.

6. The process of claim 1 wherein the organic polymer resin of step (3) is an adsorbent resin.

7. The process of claim 6 wherein the adsorbent resin is a polyacrylate, a polymethacrylate, a polystyrene cross-linked with divinyl benzene, a polyurethane or a polypropylene.

8. The process of claim 1 wherein the organic polymer resin of step (3) is an ion exchange resin, preferably an anion exchange resin, most preferably a weakly basic anion exchange resin.

9. The process of claim 1, wherein said liquid medium is an aqueous medium.

10. The process of claim 1, wherein the enzyme is a lipase.

11. The process of claim 10, wherein the lipase is derived from a strain of the genus Humicola (also known as Thermomyces), Pseudomonas, Candida, or Rhizomucor, preferably the species H. lanuginosa (also known as Thermomyces lanuginosa), C. antarctica or R. miehei.

12. A process for enzymatic modification of an organic compound comprising contacting in a reaction medium essentially devoid of free water said organic compound with an immobilized enzyme produced by the process of any of the claims 1-11.

13. The process according to claim 12, wherein the modification is a trans-esterification reaction comprising contacting a first reactant which is a fatty acid ester, a second reactant which is another fatty acid ester, an alcohol or a free fatty acid with the immobilized lipase.

14. The process of claim 13, wherein the first reactant is a triglyceride.

15. The process of claims 13 and 14, wherein the second reactant is a fatty acid ester, and the lipase positionally specific.

16. The process of claim 13, wherein the first and the second reactants are different triglycerides or different mixtures of triglycerides, and the lipase is positionally 1,3-specific.

17. The process of claim 13-16, wherein the reaction medium consists essentially of triglycerides.

18. The process of claim 13-16, wherein the reaction medium comprises an organic solvent.

19. The process of claim 1, wherein the particulate porous carrier has an average pore size of 10-500 nm.

**Patentansprüche**

1. Verfahren zur Herstellung einer Zubereitung eines immobilisierten Enzyms zur Verwendung in einem hauptsächlich organischen Medium ohne freies Wasser, umfassend:

(a) Fluidisieren eines teilchenförmigen porösen Trägers mit einer Oberfläche von 20 - 1000 $m^2/g$, einer Teilchengröße von 200 - 1000µm und umfassend ein Material, ausgewählt aus:

(1) einem anorganischen Material, das im Wesentlichen in hydrophilen oder hydrophoben Flüssigkeiten oder Gemischen davon unlöslich ist und eine hydrophile Oberfläche aufweist,

(2) einem anorganischen Material, das im Wesentlichen in hydrophilen oder hydrophoben Flüssigkeiten oder Gemischen davon unlöslich und mit organischen Einheiten beschichtet ist, so dass eine im Wesentlichen hydrophobe Oberfläche bereitgestellt wird, oder
(3) einem organischen Polymerharz,

in einer Wirbelschicht,
(b) Einbringen eines enzymthaltigen flüssigen Mediums durch Zerstäubung in das Fluidbett, so dass das Enzym auf dem Träger fixiert wird, und
(c) Entfernen von flüchtigen Bestandteilen des flüssigen Mediums von dem Träger in der Wirbelschicht.

**2.** Verfahren zur Herstellung einer Zubereitung eines immobilisierten Enzyms zur Verwendung in einem hauptsächlich organischen Medium ohne freies Wasser, umfassend:

oder hydrophoben Flüssigkeiten oder Gemischen davon unlöslich ist, mit einem Oberflächenbereich von 20 - 1000 $m^2/g$, einer Teilchengröße von 200 - 1000 μm und einer hydrophoben Oberfläche, so dass das Enzym auf dem Träger adsorbiert wird, und
(b) Einbringen einer hygroskopischen Substanz, so dass die Agglomeration des Trägers durch Absorbieren von überschüssiger Flüssigkeit unterdrückt wird, und
(c) Entfernen von flüchtigen Bestandteilen des flüssigen Mediums und der hygroskopischen Substanz aus dem Produkt in einer Wirbelschicht.

**3.** Verfahren nach Anspruch 1, wobei die Flüssigkeit in solch einer Menge eingebracht wird, dass im Wesentlichen keine Agglomeration des Trägers auftritt.

**4.** Verfahren nach Anspruch 1, wobei das anorganische Material von Schritt (1) ausgewählt ist aus der Gruppe, bestehend aus Siliciumdioxiden, Zeolithen, Aluminiumoxiden und Kaolinen.

**5.** Verfahren nach Anspruch 1, wobei das anorganische Material von Schritt (2) ausgewählt ist aus der Gruppe, bestehend aus Siliciumdioxiden, Zeolithen, Aluminiumoxiden und Kaolinen, die mit organischen Einheiten beschichtet sind, um eine hydrophobe Oberfläche bereitzustellen.

**6.** Verfahren nach Anspruch 1, wobei das organische Polymerharz von Schritt (3) ein Adsorptionsharz ist.

**7.** Verfahren nach Anspruch 6, wobei das Adsorptionsharz ein Polyacrylat, ein Polymethacrylat, ein mit Divinylbenzol vernetztes Polystyrol, ein Polyurethan oder ein Polypropylen ist.

**8.** Verfahren nach Anspruch 1, wobei das organische Polymerharz von Schritt (3) ein Ionenaustauscherharz, vorzugsweise ein Anionenaustauschharz, besonders bevorzugt ein schwach basisches Anionenaustauschharz ist.

**9.** Verfahren nach Anspruch 1, wobei das flüssige Medium ein wässriges Medium ist.

**10.** Verfahren nach Anspruch 1, wobei das Enzym eine Lipase ist.

**11.** Verfahren nach Anspruch 10, wobei die Lipase von einem Stamm der Gattung Humicola (auch bekannt als Thermomyces), Pseudomonas, Candida oder Rhizomucor, vorzugsweise der Spezies H. lanuginosa (auch bekannt als Thermomyces lanuginosa), C. antarctica oder R. miehi abgeleitet ist.

**12.** Verfahren zur enzymatischen Modifizierung einer organischen Verbindung, umfassend das In-Kontakt-Bringen der organischen Verbindung mit einem immobilisierten Enzym, das durch das Verfahren nach einem der Ansprüche 1 - 11 hergestellt wurde, in einem Reaktionsmedium im Wesentlichen ohne freies Wasser.

**13.** Verfahren nach Anspruch 12, wobei die Modifizierung eine Umesterungsreaktion, umfassend das In-Kontakt-Bringen eines ersten Reaktanten, der ein Fettsäureester ist, eines zweiten Reaktanten, der ein anderer Fettsäureester ist, eines Alkohols oder einer freien Fettsäure mit der immobilisierten Lipase, ist.

**14.** Verfahren nach Anspruch 13, wobei der erste Reaktant ein Triglycerid ist.

**15.** Verfahren nach den Ansprüchen 13 und 14, wobei der zweite Reaktant ein Fettsäureester und die Lipase positionell

spezifisch ist.

16. Verfahren nach Anspruch 13, wobei der erste und der zweite Reaktant verschiedene Triglyceride oder verschiedene Gemische von Triglyceriden sind und die Lipase positionell 1,3-spezifisch ist.

17. Verfahren nach den Ansprüchen 13 - 16, wobei das Reaktionsmedium im Wesentlichen aus Triglyceriden besteht.

18. Verfahren nach den Ansprüchen 13 - 16, wobei das Reaktionsmedium ein organisches Lösungsmittel umfasst.

19. Verfahren nach Anspruch 1, wobei der teilchenförmige poröse Träger eine mittlere Porengröße von 10 - 500 nm aufweist.

**Revendications**

1. Procédé pour produire une préparation enzymatique immobilisée à utiliser dans un milieu principalement organique et exempt d'eau libre, comprenant :

   a) la fluidisation d'un support poreux particulaire ayant une aire spécifique de 20 à 1000 $m^2$/g, une granulométrie de 200 à 100 µm et comprenant un matériau choisi parmi :

      (1) un matériau minéral, qui est pratiquement insoluble dans les liquides hydrophiles ou hydrophobes ou leurs mélanges, ayant une surface hydrophile,
      (2) un matériau minéral, qui est pratiquement insoluble dans les liquides hydrophiles ou hydrophobes ou leurs mélanges, revêtu de fragments organiques de façon à former une surface sensiblement hydrophobe, ou
      (3) une résine polymère organique,

   dans un lit fluide,
   b) l'introduction d'un milieu liquide contenant une enzyme, par atomisation dans le lit fluide, de façon à fixer l'enzyme sur le support, et
   c) l'élimination des composants volatils du milieu liquide sur le support dans le lit fluidisé.

2. Procédé pour produire une préparation enzymatique immobilisée à utiliser dans un milieu principalement organique et exempt d'eau libre, comprenant :

   a) la mise en contact d'un milieu liquide contenant une enzyme avec un support poreux particulaire qui est pratiquement insoluble dans les liquides hydrophiles ou hydrophobes ou leurs mélanges, ayant une aire spécifique de 20 à 1000 $m^2$/g, une granulométrie de 200 à 100 µm et une surface hydrophobe, de façon à adsorber l'enzyme sur le support, et
   b) l'introduction d'une substance hygroscopique de façon à supprimer l'agglomération du support par absorption du liquide en excès, et
   c) l'élimination des composants volatils du milieu liquide et de la substance hygroscopique sur ledit produit dans un lit fluidisé.

3. Procédé selon la revendication 1, dans lequel le liquide est introduit en une quantité telle qu'il ne se produise pratiquement pas d'agglomération du support.

4. Procédé selon la revendication 1, dans lequel le matériau minéral de l'étape (1) est choisi dans l'ensemble comprenant les silices, les zéolites, les alumines et les kaolins.

5. Procédé selon la revendication 1, dans lequel le matériau minéral de l'étape (2) est choisi dans l'ensemble constitué par les silices, les zéolites, les alumines et les kaolins revêtus de fragments organiques de façon à proposer une surface hydrophobe.

6. Procédé selon la revendication 1, dans lequel la résine polymère organique de l'étape (3) est une résine adsorbante.

7. Procédé selon la revendication 6, dans lequel la résine adsorbante est un polyacrylate, un polyméthacrylate, un polystyrène réticulé avec du divinylbenzène, un polyuréthane ou un polypropylène.

8. Procédé selon la revendication 6, dans lequel la résine polymère organique de l'étape (3) est une résine échangeuse d'ions, de préférence une résine échangeuse d'anions, tout spécialement une résine échangeuse d'anions faiblement basique.

9. Procédé selon la revendication 1, dans lequel ledit milieu liquide est un milieu aqueux.

10. Procédé selon la revendication 1, dans lequel l'enzyme est une lipase.

11. Procédé selon la revendication 10, dans lequel la lipase dérive d'une souche du genre Humicola (également connu sous le nom de Thermomyces), Pseudomonas, Candida ou Rhizomucor, de préférence l'espèce H. lanuginosa (également connue sous le nom de Thermomyces lanuginosa), C. antarctica ou R. miehei.

12. Procédé pour la modification enzymatique d'un composé organique, comprenant la mise en contact, dans un milieu réactionnel pratiquement exempt d'eau libre, dudit composé organique avec une enzyme immobilisée produite par le procédé de l'une quelconque des revendications 1 à 11.

13. Procédé selon la revendication 12, dans lequel la modification est une réaction de trans-estérification comprenant la mise en contact d'un premier réactif qui est un ester d'acide gras, d'un deuxième réactif qui est un autre ester d'acide gras, d'un alcool ou d'un acide gras libre avec la lipase immobilisée.

14. Procédé selon la revendication 13, dans lequel le premier réactif est un triglycéride.

15. Procédé selon les revendications 13 et 14, dans lequel le deuxième réactif est un ester d'acide gras, et la lipase est à spécificité de position.

16. Procédé selon la revendication 13, dans lequel les premier et deuxième réactifs sont des triglycérides différents ou des mélanges différents de triglycérides, et la lipase est à spécificité de position 1,3.

17. Procédé selon les revendications 13 à 16, dans lequel le milieu réactionnel est constitué essentiellement de triglycérides.

18. Procédé selon les revendications 13 à 16, dans lequel le milieu réactionnel comprend un solvant organique.

19. Procédé selon la revendication 1, dans lequel le support poreux particulaire a une taille de pores moyenne de 10 à 500 nm.

Fig. 1: Acidolysis

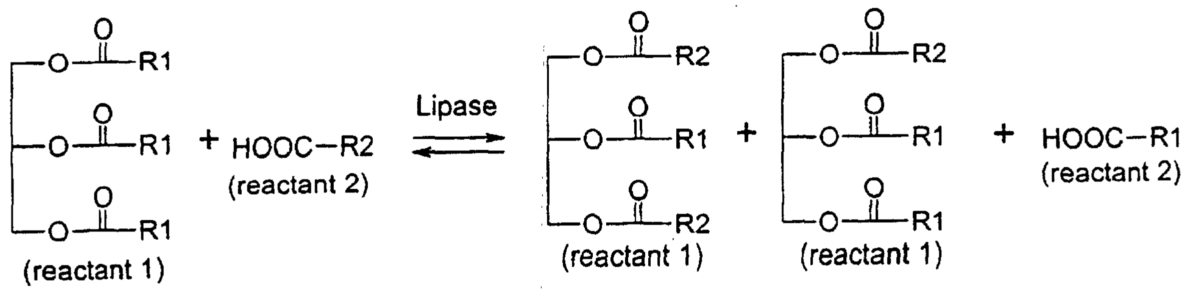


Fig. 2: Inter-esterification

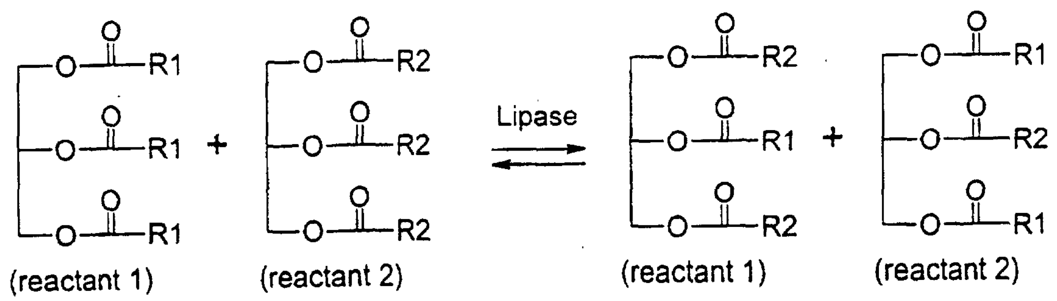


Fig. 3: Alcoholysis